(19) Europäisches Patentamt
European Patent Office
Office européen des brevets

(11) **EP 1 737 464 B1**

(12) **EUROPEAN PATENT SPECIFICATION**

(45) Date of publication and mention of the grant of the patent:
**23.07.2008 Bulletin 2008/30**

(51) Int Cl.:
***A61K 31/515*** (2006.01)     ***A61K 31/724*** (2006.01)
***A61P 11/00*** (2006.01)

(21) Application number: **05734016.8**

(22) Date of filing: **31.03.2005**

(86) International application number:
**PCT/EP2005/003345**

(87) International publication number:
**WO 2005/097133 (20.10.2005 Gazette 2005/42)**

(54) **USE OF A TRIOXOPYRIMIDINE FOR THE TREATMENT AND PREVENTION OF BRONCHIAL INFLAMMATORY DISEASES**

VERWENDUNG VON TRIOXOPYRIMIDIN ZUR BEHANDLUNG UND PRÄVENTION VON ENTZÜNDLICHEN ERKRANKUNGEN DER BRONCHIEN

UTILISATION D'UNE TRIOXOPYRIMIDINE POUR LE TRAITEMENT ET LA PR VENTION DE MALADIES INFLAMMATOIRES BRONCHIQUES

(84) Designated Contracting States:
**AT BE BG CH CY CZ DE DK EE ES FI FR GB GR HU IE IS IT LI LT LU MC NL PL PT RO SE SI SK TR**

(30) Priority: **01.04.2004 EP 04007920**

(43) Date of publication of application:
**03.01.2007 Bulletin 2007/01**

(73) Proprietors:
• **F. Hoffmann-La Roche AG**
  **4070 Basel (CH)**
• **Université de Liège**
  **4031 Angleur (BE)**

(72) Inventors:
• **CATALDO, Didier**
  **B-4870 Trooz (BE)**
• **DELATTRE, Luc**
  **B-4680 Hermee (BE)**
• **EVRARD, Brigitte**
  **B-4537 Verlaine (BE)**
• **FOIDART, Jean-Michel**
  **B-4870 Trooz (BE)**
• **KRELL, Hans-Willi**
  **82377 Penzberg (DE)**

(74) Representative: **Schreiner, Siegfried Roche Diagnostics GmbH, Patent Department Pharma (TR-E), P.O. Box 1152 82372 Penzberg (DE)**

(56) References cited:
**WO-A-01/25217        WO-A-02/34726
WO-A-02/34753        WO-A-97/23465
WO-A-98/58925**

• **GRAMS F ET AL: "PYRIMIDINE-2,4,6-TRIONES: A NEW EFFECTIVE AND SELECTIVE CLASS OF MATRIX METALLOPROTEINASE INHIBITORS" BIOLOGICAL CHEMISTRY, vol. 382, no. 8, August 2001 (2001-08), pages 1277-1285, XP008009641 ISSN: 1431-6730**
• **SAFAK O ET AL: "LOKALE HEMMUNG DER MATRIXMETALLOPROTEINASEAKTIVITAET ZUR VERBESSERUNG DER VERZOEGERTEN WUNDHEILUNG IM TIERMODELL ENHANCEMENT OF IMPAIRED WOUND HEALING IN AN ANIMAL MODEL BY LOCAL INHIBITION OF MATRIX METALLOPROTEINASE ACTIVITY" CHIRURGISCHES FORUM FUER EXPERIMENTELLE UND KLINISCHE FORSCHUNG, BERLIN, DE, 2003, pages 229-231, XP009034656 ISSN: 0303-6227**

**Description**

The present invention relates to the use of a trioxopyrimidine compound for the treatment and prevention of bronchial inflammatory diseases.

Introduction

Matrix metalloproteases (MMPs) are a family of zinc- and calcium-dependent proteases that are capable of degrading the extracellular matrix (ECM) and basement membrane (Egeblad, M., and Werb, Z., Nat. Rev. Cancer 2 (2002) 161-174; Overall, C.M., and Lopez-Otin, C., Nat. Rev. Cancer 2 (2002) 657-672). They are believed to have pivotal roles in embryonic development and growth (Holmbeck, K., et al., Cell 99 (1999) 81-92; Vu, T.H., et al., Cell 93 (1998) 411-422) as well as in tissue remodeling and repair (Shapiro, S.D., Curr. Opin. Cell Biol. 10 (1998) 602-608; Lund, L.R., et al., EMBO J. 18 (1999) 4645-4656). Excessive or inappropriate expression of MMPs may therefore contribute to the pathogenesis of many tissue-destructive processes, including tumor progression (Egeblad, M., and Werb, Z., Nat. Rev. Cancer 2 (2002) 161-174; Overall, C.M., and Lopez-Otin, C., Nat. Rev. Cancer 2 (2002) 657-672) and aneurysm formation (Carmeliet, P., et al., Nat. Genet. 17 (1997) 439-444). MMP effects are far from being restricted to ECM degradation (Chang, C., and Werb, D., Trends Cell Biol. 11 (2001) S37-43). Peptide growth factors that are sequestered by ECM proteins become available once degraded by MMP-9 (Manes, S., et al., J. Biol. Chem. 274 (1999) 6935-6945). MMPs can increase the bioavailability of VEGF (Bergers, G., et al., Nat. Cell Biol. 2 (2000) 737-744) but also generate angiogenesis inhibitors such as angiostatin by cleavage of plasminogen (Dong, Z., et al., Cell 88 (1997) 801-810).

Inhibition of MMPs, either with the naturally occurring Tissue Inhibitors of Metalloproteases (TIMPs), or with low molecular weight inhibitors, resulted in impressive anti-tumor and anti-metastatic effects in animal models (Brown, P.D., Med. Oncol. 14 (1997) 1-10). Most of the low-molecular weight inhibitors of MMPs are derived from the hydroxamic acid compound class and inhibit MMPs in a broad manner, being not selective for MMP-2 and MMP-9, the key MMPs in tumor invasion, metastatic spread, and angiogenesis. However, MMP inhibiting molecules from another structural class, the trioxopyrimidines, have been described, e.g. in WO 97/23465 and WO 01/25217. This class of compounds is extremely potent, and highly selective, with an almost exclusive specificity for MMP-2, MMP-9, while sparing most other members of the MMP family of proteases.

Several MMP inhibitors, predominantly of the hydroxamic acid substance class with broad substrate specificity were, and in part still are, in clinical testing for anti-tumor treatment. All of the published clinical results with these inhibitors were disappointing, showing little or no clinical efficacy (Fletcher, L., Nat. Biotechnol. 18 (2000) 1138-1139). The reason for this lack of efficacy in the clinic most likely is the fact that patients could not be given high enough doses for anti-tumor or anti-metastatic activity because of the side effects associated with these broadly acting inhibitors. These dose-limiting side effects were predominantly arthralgias and myalgias (Drummond, A.H., et al., Ann. N.Y. Acad. Sci. 878 (1999) 228-235). As a possible way to circumvent this problem, the combination of MMP inhibitors with classical cytostatic/cytotoxic compounds was evaluated in animal studies. Indeed, in these experiments, MMP inhibitors, in combination with cytostatic/cytotoxic drugs, showed enhanced tumor inhibiting efficacy (Giavazzi, R., et al., Clin. Cancer Res. 4 (1998) 985-992). In addition, International Patent Application No. PCT/EP02/04744 shows the combination of trioxopyrimidine based gelatinase inhibitors and cytotoxic/cytostatic compounds such as cisplatin, Paclitaxel, Gemcitabine or Etoposide.

There have been made a lot of attempts to identify compounds which prevent or inhibit bronchial inflammatory diseases. Inhaled synthetic glucocorticosteroids are widely used in the treatment of bronchial asthma where they provide very effective first line treatment. However, a range of unwanted side effects and the often complex dosing schedules associated with these drugs frequently result in poor patient compliance. Loteprednol etabonate, an inactive metabolite soft steroid, is being examined in clinical trials for its effects on airway inflammation (Szelenyi, I., et al., Drugs Today 36 (2000) 313-320). Ciclesonide, a pro-drug soft steroid, has demonstrated efficacy without side effects in a once daily formulation in asthma patients and is being developed for the treatment of both asthma and chronic obstructive pulmonary disease (Rohatagi, S., et al., J. Clin. Pharmacol. 43 (2003) 365-378).

Therefore, there exists a need for highly potent substances which can be used for the treatment or prevention of bronchial inflammatory diseases.

Description of the Invention

It was surprisingly found that trioxopyrimidine-based MMP inhibitors which are highly selective for MMP-2, MMP-9 and MMP-14 are useful for the treatment or prevention of bronchial inflammatory diseases.

The invention therefore provides the use of a trioxopyrimidine compound having an inhibitory activity against MMP-1, MMP-2, MMP-3, MMP-9 and MMP-14 defined as

a) an $IC_{50}$ value of less than 5 μM for MMP-2, MMP-9 and MMP-14 each;

b) a ratio of more than 100 for the $IC_{50}$ values of MMP-1:MMP-2, MMP-1: MMP-9, MMP-1:MMP-14; and

c) a ratio of more than 10 for the $IC_{50}$ values of MMP-3:MMP-2, MMP-3: MMP-9, MMP-3:MMP-14,

for the treatment of prevention of bronchial inflammatory diseases in a host mammal in need of such treatment.

$IC_{50}$ values are measured by an in vitro assay for MMP enzymatic activity. Such an assay is described by Stack, M.S., and Gray, R.D., J. Biol. Chem. 264 (1989) 4277-4281. This assay is based on the determination of MMP enzymatic activity on a dinitrophenol substrate and fluorescence measurement of the substrate after cleaving by MMPs.

The invention further provides the use of such trioxopyrimidine compounds for the manufacturing of a medicament for the treatment of bronchial inflammatory diseases in a patient suffering from such a disease.

Matrix metalloproteinases are well-known in the state of the art and are defined, e.g., by their EC numbers (MMP-1 EC 3.4.24.7; MMP-2 EC 3.4.24.24; MMP-3 EC 3.4.24.17, MMP-9 EC 3.4.24.35, MMP-14 EC 3.4.24).

Trioxopyrimidines useful for the invention are compounds from a well-known structural class. Such compounds are described in, for example, US Patent Nos. 6,242,455 and 6,110,924; WO 97/23465, WO 98/58915, WO 01/25217, and Grams, F., et al., Biol. Chem. 382 (2001) 1277-1285, and are effective and highly selective for MMP-2, MMP-9, and MMP-14.

The compounds to be used according to the invention, are:

5-Biphenyl-4-yl-5-[4-(4-nitro-phenyl)-piperazin-1-yl]pyrimidine-2,4,6-trione (Compound I)

5-(4-Phenoxy-phenyl)-5-(4-pyrimidin-2-yl-piperazin-1-yl)-pyrimidine-2,4,6-trione (Compound II)

5- [4-(4-Chloro-phenoxy)-phenyl] -5-(4-pyrimidin-2-yl-piperazin-1-yl)-pyrimidine-2,4,6-trione (Compound III)

5- [4-(3,4-Dichloro-phenoxy)-phenyl] -5-(4-pyrimidin-2-yl-piperazin-1-yl)-pyrimidine-2,4,6-trione (Compound IV)

5- [4-(4-Bromo-phenoxy)-phenyl] -5-(4-pyrimidin-2-yl-piperazin-1-yl)-pyrimidine-2,4,6-trione (Compound V).

According to the invention the trioxopyrimidine-based inhibitors have to be administered to the patient suffering from such a disease, over several months or years (especially in case of prevention), to the patient in need of such a therapy. The trioxopyrimidine compounds are administered preferably as sprays, with non-toxic doses ranging between micro and nanomolar concentrations.

The invention relates to a use for treating bronchial inflammatory diseases, preferably asthma and chronic obstructive pulmonary disease (COPD) in a host mammal in need of such treatment, e.g., a patient suffering from such a disease, by the application of a trioxopyrimidine compound according to the invention to a patient in a pharmaceutically effective amount. Asthma is an inflammatory disease of the bronchial tree related or not to an allergen exposure. This inflammation provokes symptoms in patients by stimulating the bronchial smooth muscles to contract, enhancing the mucus secretion, and inducing bronchial morphological changes thought to be an aggravating factor regarding the course of the disease. Airway hyperresponsiveness is a hallmark of the disease and is responsible for most of symptoms. Bronchial tree is a very complex tissue with many cell types (epithelial cells, smooth muscle cells, inflammatory cells, nerves, mucus producing cells, fibroblasts, and the like) and the bronchial remodelling events which comprise many aspects mainly consist in a deposition of extracellular matrix components in the bronchial walls and an hyperplasia of the mucus producing cells. The use of trioxopyrimidine compounds according to the invention inhibits the inflammatory cells influx in the compartments of bronchoalveolar lavage and peribronchial tissue and inhibits the hyperresponsiveness defined as an abnormal response to stimulating agents such as methacholine. The disease and current treatments are reviewed in, e.g., GINA Workshop Report, Global Strategy for Asthma Management and Prevention (NIH Publication No. 02-3659) and Fabbri, L.M., and Hurd, S.S., Eur. Respir. J. 22 (2003) 1-2.

The invention therefore further relates to a use for the treatment of bronchial inflammatory diseases in a patient suffering from such a disease, using a trioxopyrimidine compound according to the invention in a therapeutically effective amount.

The invention preferably further relates to a use for the treatment of emphysema in a patient suffering from such a disease, using trioxopyrimidine compounds according to the invention. In such a disease, the alveolar walls are destroyed by proteolytic processes and this destruction impairs the transfer of oxygen to the blood. Physiological problems also occurs because of the derived hyperinflation which causes abnormalities in the ventilation by causing a dysfunction of respiratory muscles and because of a hypertension in pulmonary arteries leading to cardiac failure in advanced stages.

According to the invention the trioxopyrimidine compound has to be administered over several months or years, to the patient in need of such a therapy. The trioxopyrimidine compounds are administered preferably by the aerosolization of a liquid or powder formulation, with non-toxic doses ranging between micro and nanomolar concentrations per kg and day.

The exact dosage of the MMP inhibitors will vary, but can be easily determined. In general, the preferred daily dosage of the inhibitors will range between 1 $\mu$mol/kg and day to 100 nmol/kg and day.

The pharmaceutical compositions are aqueous compositions having physiological compatibility. The compositions include, in addition, auxiliary substances, buffers, preservatives, solvents and/or viscosity modulating agents. Appropriate buffer systems are based on sodium phosphate, sodium acetate or sodium borate. Preservatives are required to prevent microbial contamination of the pharmaceutical composition during use. Suitable preservatives are, for example, benzalkonium chloride, chlorobutanol, methylparabene, propylparabene, phenylethyl alcohol, sorbic acid. Such preservatives are used typically in an amount of 0.01 to 1% weight/volume.

Suitable auxiliary substances and pharmaceutical formulations are described in Remington's Pharmaceutical Sciences, 16th ed., 1980, Mack Publishing Co., edited by Oslo et al. Typically, an appropriate amount of a pharmaceutically-acceptable salt is used in the formulation to render the formulation isotonic. Examples of a pharmaceutically acceptable substances include saline, Ringer's solution and dextrose solution. The pH of the solution is preferably from about 5 to about 8, and more preferably from about 7 to about 7.5.

A further preferred object of the invention is a pharmaceutical composition of a trioxopyrimidine according to the invention for the treatment of bronchial inflammatory diseases, and its use, containing a trioxopyrimidine-cyclodextrin complex (inclusion of the trioxopyrimidine in the cyclodextrin) formed of such a trioxopyrimidine or a salt thereof and a cyclodextrin, preferably a water-soluble cyclodextrin derivative (water soluble being defined as a solubility of at least 0.5 g/100ml water at 25°C). In the complex, preferably 1 mol of trioxopyrimidine is complexed and enclosed by 1 mol of $\alpha$, $\beta$- or $\gamma$-cyclodextrin or a derivative thereof. The preferred cyclodextrins are

- alpha-cyclodextrin and its synthetic derivatives such as HP$\alpha$CD, methylated $\alpha$CD, hydroxybutyl $\alpha$CD, maltosyl $\alpha$CD, glucosyl $\alpha$CD.
- beta-cyclodextrin and its synthetic derivatives such as HP$\beta$CD, SBE$\beta$CD, RM$\beta$CD, DIME$\beta$CD, TRIME$\beta$CD, hydroxybutyl $\beta$CD, glucosyl $\beta$CD, maltosyl $\beta$CD.
- gamma-cyclodextrin and its synthetic derivatives such as HP$\gamma$CD, RM$\gamma$CD and DIME$\gamma$CD, hydroxybutyl $\gamma$CD, glucosyl $\gamma$CD, maltosyl $\gamma$CD.

Cyclodextrins useful according to the invention are cyclic oligosaccharides produced by enzymatic degradation of starch, which are composed of a variable number of glucopyrannose units, mostly 6, 7 or 8: these cyclodextrins are respectively named $\alpha$, $\beta$, and $\gamma$ cyclodextrins ($\alpha$CD, $\beta$CD and $\gamma$CD). Cyclodextrins according to the invention are cyclodextrins per se or cyclodextrin derivatives, which are at least water soluble in an amount of 0.5 gr/100rnl at 25°C.

The water-soluble cyclodextrin derivative preferably used in the present invention refers to a derivative having water solubility of at least that of $\beta$-cyclodextrin. Examples of such water-soluble cyclodextrin derivatives are sulfobutylcyclodextrin, hydroxypropylcyclodextrin, maltosylcyclodextrin, and salts thereof. In particular, sulfobutyl-$\beta$-cyclodextrin, hydroxypropyl-$\beta$-cyclodextrin, maltosyl-$\beta$-cyclodextrin, and salts thereof

Derivatives preferred according to the invention are also methylcyclodextrins (products of the cyclodextrins methylation), dimethylcyclodextrins (DIMEB) (preferably substituted in 2 and in 6), trimethylcyclodextrins (preferably substituted in 2, 3 and 6), "random methylated" cyclodextrins (RAMEB) (preferably substituted at random in 2, 3 and 6, but with a number of 1,7 to 1,9 methyl by unit glucopyrannose), hydroxypropylcyclodextrins (HPCD, hydroxypropylated cyclodextrins preferably substituted randomly mainly in position 2 and 3 (HP-$\beta$CD, HP-$\gamma$CD)), sulfobutylethercyclodextrins (SBECD), hydroxyethylcyclodextrins, carboxymethylethylcyclodextrins, ethylcyclodextrins, cyclodextrins amphiphiles obtained by grafting hydrocarbonated chains in the hydroxyl groups and being able to form nanoparticles, cholesterol cyclodextrins and triglyceridescyclodextrins obtained by grafting cyclodextrins monoaminated (with a spacer arm).

The trioxopyrimidine-cyclodextrin complex of the present invention may be obtained by producing an aqueous solution containing a trioxopyrimidine compound thereof and a water-soluble cyclodextrin derivative. The water-soluble cyclodextrin derivative is used in an amount of preferably one mol or more per one mol trioxopyrimidine, more preferably 1-10 mol, and particularly preferably 1-2 mol cyclodextrin per mol trioxopyrimidine.

The higher the concentration of the water-soluble cyclodextrin derivative, the more the solubility of the trioxopyrimidine increases. No particular limitation is imposed on the method for producing the aqueous solution, and for example it is produced by use of water or a buffer in a temperature range approximately from -5 to 35° C.

When a cyclodextrins aqueous solution is stirred with an excess of a trioxopyrimidine, there is a complex formation between these two molecules. The filtration of this solution allows recovering the complex a trioxopyrimidine in solution in the filtrate, the complex being soluble in water. The complex can also be obtained by mixing a solubilized known quantity of a trioxopyrimidine in aqueous solution with a solubilized known quantity of CD by calculating the adequate proportions.

Another way of obtaining a complex is to add a solution of a trioxopyrimidine in a solvent (e. g. alcohol, acetone, etc) to a cyclodextrin aqueous solution. The complex can be formed after sufficient stirring, either after evaporation of the solvent, or even in the presence of the solvent.

The lyophilization or the nebulization of solutions of the complex according to the invention allows the complex to be obtained in solid form. One can thus obtain a complex in the form of an amorphous powder. It is also possible to obtain the complex in the solid state after dissolution of CD and a trioxopyrimidine in an appropriate organic solvent and further evaporation of the solvent.

Other methods can be used for solid complexes preparation which are violent stirring of a suspension of a trioxopyrimidine and CD in a very small quantity of water, then complex collecting after drying or the use of $CO_2$ in a supercritical state for mixing a trioxopyrimidine and CD in presence of $CO_2$ in a supercritical state.

The complex according to the present invention can be prepared, for example, in a manner known per se from a solution or using the paste method, where the weight ratio of cyclodextrin to trioxopyrimidine must be between 2 to 540, and is preferably between 2 to 25, particularly preferably in the region of 2.6 to 3.5 (for a 1:1 complex with cyclodextrin) or of 5.2 to 6.2 (for a 1:2 complex with cyclodextrin).

It is preferred to prepare the complex from a concentrated, aqueous cyclodextrin preparation. The cyclodextrin concentration of the preparation is preferably between 50 and 400 mM. Preference is given to a cyclodextrin concentration of from 100 to 250 nM. Depending on the consistency, the mixtures are intensively stirred or kneaded. The percent by weight of the cyclodextrin is based upon the total weight of the aqueous cyclodextrin preparation.

The reaction temperature is usually between 20° C and 80° C, preferably between 20° C and 60° C, particularly preferably between 25° C and 45° C. The reaction time depends on the temperature and is between one hour and some days. Preference is given to a reaction time of at least 7 days to reach equilibrium of complex formation.

According to the invention it has been established that complexes between a trioxopyrimidine and a cyclodextrin increase the solubility of a trioxopyrimidine in water amazingly. It is also apparent that the formation of the complex did not interfere with the pharmacological properties of the trioxopyrimidine.

All these properties allow to prepare liquid formulations as solutions for injection or for nebulization and allow the bioavailability to be improved, in particular orally. A trioxopyrimidine-cyclodextrin complex of the present invention may be used as such or in a powder form which is obtained by removing co-existing water. Examples of the method for removing water include lyophilization and drying under reduced pressure. A powder product obtained from lyophilization is particularly preferred.

The trioxopyrimidine-cyclodextrin complex of the present invention exhibits its effects through either oral administration or parenteral administration, and it is preferably formed into a formulation for parenteral administration, particularly an injection formulation or topical administration, particularly an aerosol formulation.

Examples of the form of formulation include tablets, capsules, powders, and granules. These may be produced through a known technique by use of typical additives such as excipients, lubricants, and binders.

A preferred pharmaceutical formulation for nebulization contains trioxopyrimidine, cyclodextrin (CD), NaCl and water. Especially preferred is a combination of (for 200 ml of solution):

Trioxopyrimidine 0.05-0.2 g, preferably 0.1 g; 10-50 g CD, preferably 20 g CD, preferably HPβCD; sodium chloride 1.2-1.5 g, preferably 1.42 g (isotonicity) and water, preferably pyrogen-free, sterile, purified water ad 200 ml.

Such a formulation is useful for the treatment of bronchial inflammatory diseases.

The solution is prepared by dissolving CD in 100 ml of purified water, adding trioxopyrimidine and NaCl by stirring so as to dissolve them and complete with water so as to obtain 200 ml of solution. Preferably the solution is sterilized by filtration through a 0.22 $\mu$m polypropylene membrane or by a steam sterilization process.

The following examples, references, and figures are provided to aid the understanding of the present invention.

## Description of the Figures

**Figure 1**  Effects of intraperitoneal injection of a Compound Isuspension on BAL eosinophil counts (Fig la) and peribronchial inflammation score (Fig 1b). Controls are mice exposed only to PBS and not allergen (PBS) and mice exposed to ova by inhalation and placebo by intraperitoneal injection (OVA)

**Figure 2**  Therapeutic effects of Compound I-HP-β-CD complex, fluticasone and placebo (PLAC) administered by aerosols on BAL eosinophilia (2a), peribronchial inflammation score (2b), and tissue eosinophils infiltration score (2c) in a short term (5 days) allergen exposure model.

**Figure 3**  Therapeutic effects of Compound I-HP-β-CD complex, fluticasone and placebo (PBS) administered by aerosols on BAL eosinophilia (3a), peribronchial inflammation score (3b), and tissue eosinophils infiltration score (3c) in a long term (11 weeks) allergen exposure model. Mice sensitized but unexposed to allergens (PBS) and mice sensitized and exposed to OVA (PLAC) were treated by PBS inhalation.

**Figure 4**     Phase solubility diagram of Compound I with HP-β-CD in purified water (•), L-lysine 50 mM (x) or L-lysine 500 mM (▲).

**Figure 5**     Mean (± S.D.) Compound I serum concentration (a) or logarithm of the mean Compound I serum concentration (b) versus time curve after intravenous administration (5 mg/kg) to sheep (n = 6).

**Figure 6**     Mean (± S.D.) Compound I serum concentration (a) or logarithm of the mean Compound I serum concentration (b) versus time curve after oral administration (15 mg/kg) of a solution (▲) and a suspension (•) to sheep (n = 5 for solution and n = 6 for suspension).

**<u>Abbreviations</u>**

| CD | cyclodextrin |
|---|---|
| HPβCD or HP-β-CD | Hydroxypropyl β-cyclodextrin |
| I.V. | intravenous |
| MMP | matrix metalloprotease |
| OVA | ovalbumin |
| PBS | phosphate buffered saline β-cyclodextrin |

**<u>Example 1</u>**

**Preparation of a soluble complex of compound I and cyclodextrin (CD)**

1.1 Weigh 20 mg of compound I. Add 2 ml of solution of HPβCD 200 mM. Stir for 24 h at 37°C. Filter in Millipore filter Millex HV 0.45 μm. The solution obtained after filtration contains the complex compound I-CD in solution.

1.2. Weigh 2.5 mg of compound I. Add 2 ml of solution of HPβCD 200 mM. Stir at 37°C for 24 h or until compound I is completely dissolved. The solution obtained in this way contains the complex compound I-CD.

**<u>Example 2</u>**

**Enhancement of solubility of the soluble complex of compound I and cyclodextrin (CD) by adding L-lysine solution.**

Solubility studies were performed as described by Higuchi, T., and Connors, K.A., Advances in Analytical Chemistry and Instrumentation 4 (1965) 117-212. Excess amounts of Compound I were added to increasing concentrations of HP-β-CD (0 - 200 mM) in 5 ml dissolution media, either purified water or L-lysine solutions (50 mM or 500 mM). The glass containers were sealed and the suspensions were shaken in a water-bath at 25°C until complexation equilibrium was reached (7 days). An aliquot was filtered through a 0.45 μm PVDF membrane filter and assayed for Compound I content by a validated liquid chromatography (LC) method.

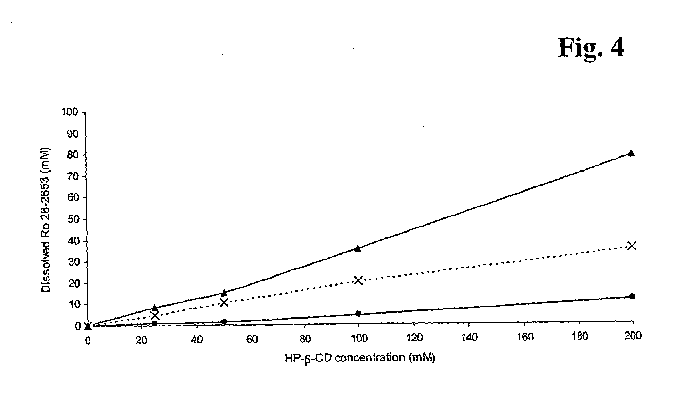

Fig. 4 shows the phase solubility diagram of Compound I obtained at 25°C in the presence of HP-β-CD in purified water, in a 50 mM L-lysine solution and in a 500 mM L-lysine solution. In the three cases, the aqueous solubility of Compound I increases as a function of CD concentration. The solubility diagram obtained in the absence of L-lysine confirms the previously mentioned results: the solubility of Compound I in a 200 mM HP-β-CD solution is about 5.5 mg/ml (11 mM) which corresponds to an approximately 10,000-fold increase of the Compound I's aqueous solubility.

In the presence of L-lysine, the Compound I solubility in HP-β-CD solutions is even much higher. The solubility in a 200 mM HP-β-CD solution is increased about 2 and 7 times in the presence of 50 mM and 500 mM of L-lysine respectively. Table 8 shows solubility data of Compound I in the different media. Results show a synergistic effect between L-lysine and HP-β-CD. The solubility in the presence of both 500 mM L-lysine and 200 mM HP-β-CD (38.14 mg/ml) is higher than that expected by adding the effect of HP-β-CD and L-lysine separately (5.53 mg/ml and 0.09 mg/ml). This synergistic effect between L-lysine and HP-β-CD allows an important increase of Compound I aqueous solubility (70,000-fold with 500 mM of L-lysine and 200 mM of HP-β-CD).

**Table 1:**

| Solubility of Compound I [mg/ml] in purified water and in L-lysine (50 mM and 500 mM) without or with HP-β-CD (200 mM) | | |
|---|---|---|
| | Solubility without CD [$\mu$g/ml] | Solubility with HP-β-CD (200 mM) [$\mu$g/ml] |
| Purified Water | 0.56 | 5530 |
| L-lysine 50 mM | 50 | 17080 |
| L-lysine 500 mM | 90 | 38140 |

## Example 3

### Determination of MMP enzymatic activity

Inhibitors were tested in a modified fluorescence-assay as described by Stack, M.S., and Gray, R.D., J. Biol. Chem. 264 (1989) 4277-4281. Human MMP-1, MMP-2, MMP-3, MMP-9 and MMP-14 are commercially available (e.g. Calbiochem). The pro-enzymes were activated with 1 mM APMA (incubation for 30 min at 37°C) immediately before testing. Activated enzyme is diluted to 100 ng/ml in incubation buffer (50 mM Tris, 100 mM NaCl, 10mM CaCl2, pH 7.6). The compounds were dissolved in 100% DMSO. For $IC_{50}$ determination a minimum of 8 dilution steps between 0.5 - 1000 nM have been prepared. DNP-substrate (Bachem M1855, 255$\mu$M) was dissolved in incubation buffer.

The test tube contains 970$\mu$l incubation buffer, 10$\mu$l inhibitor solution and 10$\mu$l enzyme solution. The reaction was started by adding the 10$\mu$l substrate solution.

Kinetics of activity were determined using excitation at 280 nm and emission at 346 nm measured on a Fluoro-Max™ (Spex Industries Inc., Edison, NJ, USA) over 120 sec. DMSO has been used as control instead of inhibitor solution.

$IC_{50}$'s are defined as the concentration of inhibitor that gives a signal that is 50% of the positive enzyme control.

$IC_{50}$ values (nM) are shown in Table 1.

**Table 2**

| | MMP-1 | MMP-2 | MMP-3 | MMP-9 | MMP-14 |
|---|---|---|---|---|---|
| Compound I [nM] | 53,000 | 65 | 3,500 | 260 | 1 |
| Batimastat [nM] | 25 | 32 | 67 | 23 | 19 |

## Example 4

### Pharmaceutical compositions

Different compositions of formulations are given for example non-exhaustively.

A preferred example for an injectable formulation is:

**-** HP-βCD 200 mM; Compound I 1 mg/ml; Sterile water for injection q.s.

For 25 ml of solution:

### a) Preparation of the solution:

Weigh 6.77 g of HPβCD (4.2 % of $H_2O$) and dissolve them in 25 ml of water by injection. Add 25 mg of compound I and heat in a water bath until the latter is completely dissolved. Sterilize the solution by filtration.

### b) Characteristics of the solution:

The solution osmolality is 308 mOs/kg. The pH is 7.2.

The concentration of compound I and/or of CD can be modified in function of the requirements. It is preferred to adjust the tonicity by addition of NaCl.

A preferred formulation for nebulization is:

For 200 ml of solution:

| | |
|---|---|
| - Compound I | 0.1 g |
| - HPβCD exempt from pyrogenic | 20.15 g (75mM) |
| - Sodium chloride | 1.42 g (isotonicity) |
| - Pyrogen-free, sterile, purified water, | q.s. ad 200 ml |

a) Weigh 20.15 g of HPβCD exempt from pyrogenic (3.2 % $H_2O$, ROQUETTE) and dissolve them in 100 ml of purified water.

b) Weigh 0.1 g of compound I, and 1.42 g of sodium chloride and add them to solution (a) by energetically stirring so as to dissolve them.

c) Complete with water so as to obtain 200 ml of solution.

d) Sterilize by filtration through a 0.22 $\mu$m polypropylene membrane.

## Example 5

**Use of formulations containing compound I and HPβCD for therapy of allergen-induced airway inflammation and bronchial hyperresponsiveness in a mouse model of asthma.**

### Materials

HP-β-CD (degree of substitution = 0.64) originates from Roquette (France). Apyrogenic phosphate buffered saline (PBS) was purchased from Bio-Wittaker (Verviers, Belgium) and methacholine from Sigma-Aldrich (Germany). All other materials were of analytical grade. Sterile water for injection was used throughout this study. Sterile, apyrogenic and isotonic CD solutions were prepared at 20, 50 and 75 mM. A commercially available fluticasone solution for inhalation (Flixotide® lmg/ml) was purchased from Glaxo-Smithkline (Genval, Belgium)

### Sensitization, allergen exposure and therapeutic protocols

In order to study the modulation of airway inflammation by intraperitoneal injection of Compound I, mice were sensitized with 10 $\mu$g ovalbumin alumin-adsorbed (aluminject, perbio, Erembodegem, Belgium) injected intraperitonealy at days 0 and 7 and were subsequently exposed to OVA 1% or PBS aerosols for 30 minutes from day 21 to 24. Intraperitoneal injections were performed 30 min before OVA inhalations. The different injected formulations were: cremophor 10%-DMSO 10%-PBS 80%- Compound 130 mg/kg (suspension); cremophor 10%-DMSO 10%-PBS 80%- Compound 13.75 mg/kg (solution); HPβCD 200 mM Compound 17.5 mg/kg (solution); HPβCD 200 mM. All results were compared to mice sensitized and exposed to PBS and OVA treated with PBS injected intraperitonealy. In order to study the modulation of airway inflammation by inhaled Compound I, mice were sensitized as described previously. Two protocols referred to as short exposure challenge and long-term exposure challenge were used. In the short exposure challenge, mice were exposed to aerosols of Compound I- complex at concentrations of 0.03 and 0.3 mg/ml of active compound in aqueous solution of from day 21 to 27 during 30 min in a Plexiglas exposure chamber (30 x 20 x 15 cm). Mice were exposed to OVA aerosols 30 minutes after the Compound I inhalation from day 23 to 27. In the so called long-term inhalation challenge, mice were exposed to aerosols of Compound Iat concentrations of 0.03 and 0.3 mg/ml complexed with HPβCD in an aqueous solution during 30 min five days odd weeks and to OVA aerosols 3 days odd weeks for 11 weeks. No inhalations were performed during even weeks.

The aerosol were produced by using an ultrasonic nebuliser SYSTAM (Systeme Assistance Medical, Le Ledat, France), the vibration frequency of which is 2.4 MHz with variable vibration intensity and ventilation levels. Vibration intensity was fixed in position 6 and the ventilation level was 25($v_{1/2}$) l/min.

### Airway responsiveness measurement

Twenty-four hours after the last allergen exposure, the bronchial hyper responsiveness was determined by measuring the Penh using a barometric plethysmograph as proposed by Hamelmann, E., et al., Am. J Respir. Crit. Care Med. 156 (1997) 766-775). The Penh was measured at baseline and 5 min after the inhalation of increasing doses (25, 50, 75 and 100 mM) of methacholine (Mch).

**Bronchoalveolar lavage (BAL) and histology**

Immediately after the assessment of airway responsiveness, mice were sacrificed and 1 ml of PBS free of ionised calcium and magnesium but supplemented with 0.05 mM sodium EDTA was instilled 4 times via a tracheal cannula and recovered by gentle manual aspiration. The recovered bronchoalveolar lavage fluid (BAL) was centrifuged (1800 rpm for 10 min at 4°C). The cell pellet was washed twice and finally resuspended in 1 ml of PBS. A total cell count was performed in a Thoma chamber and the differential cell counts on at least 400 cells were performed on cytocentrifuged preparations (Cytospin 2; Cytospin, Shandon td., Runcorn, Cheshire, U.K.) using standard morphologic criteria after staining with Diff-Quick (Dade, Germany). After BAL, the thorax was opened and the left main bronchus was clamped. The left lung was excised and frozen immediately in liquid $N_2$ for protein chemistry and mRNA extraction while the right lung was processed for histology. As previously described (Cataldo, D.D., et al, Am. J. Pathol. 161 (2002) 491-498), the right lung was infused with 4% paraformaldehyde and embedded in paraffin. Sections of 5 $\mu$m thickness from all lobes were stained with haematoxylin and eosin. The extent of peribronchial infiltrates was estimated by an inflammation score. Slides were coded and the peribronchial inflammation was graded in a blinded fashion using a reproducible scoring system described elsewhere (Cataldo, D.D., et al, Am. J. Pathol. 161 (2002) 491-498). A value from 0 to 3 per criteria was adjudged to each tissue section scored. A value of 0 was adjudged when no inflammation was detectable, a value of 1 for occasional cuffing with inflammatory cells, a value of 2 when most bronchi were surrounded by a thin layer (1 to 5 cells) of inflammatory cells and a value of 3 when most bronchi were surrounded by a thick layer (>5 cells) of inflammatory cells. As 5-7 randomly selected tissue sections per mouse were scored, inflammation scores could be expressed as a mean value per animal and could be compared between groups. Another score referred to as tissue eosinophil infiltration score, specifically reflecting the amounts of eosinophils infiltrating the bronchial walls, was measured as follows: after a congo red staining, seven bronchi were studied per mouse. The eosinophils were counted around the bronchi within the limits of the airway wall, the perimeter of the epithelial basement membrane was measured and the results were expressed as number of eosinophils/mm of basement membrane. The left lung was snap frozen in liquid nitrogen and crushed using a Mikro-Dismembrator S (Braun Biotech International, Melsungen, Germany) and the extracts stored at -80°C before studied. Kidneys were excised and paraffin embedded, sections of 5 $\mu$m were stained by haematoxylin and eosin. Blood was sampled by cardiac puncture and serum was stored at -80°C until analysis were performed.

All in vivo manipulations were approved by the local Veterinarian Ethics Committee.

**Intraperitoneal injection of Compound I**

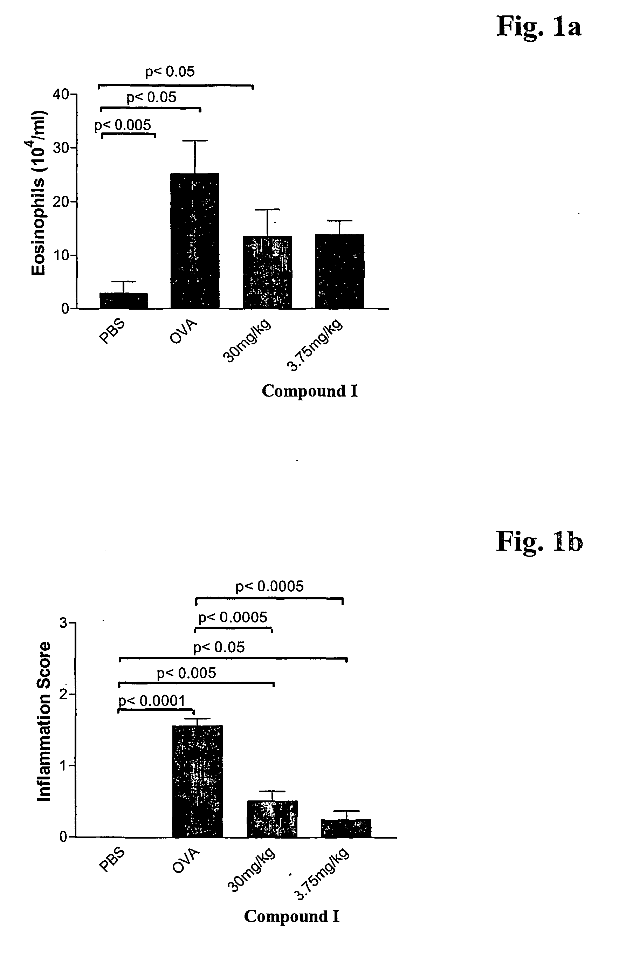

The intraperitoneal injection of Compound I (either solution or precipitate) lowered the allergen-induced airway eosinophilic inflammation in BAL at doses of 3.75 to 30 mg/kg when compared to placebo (Figure Ia). At the same doses, the peribronchial inflammation scores were also significantly lowered by Compound I with an equal efficacy of all tested formulations (Figure 1b). The tissue eosinophil infiltration score was significantly lowered by the intraperitoneal injection of Compound I at doses of 7.5 and 25 mg/kg.

**Inhalational exposure to Compound I and Compound I- HP$\beta$CD complexes.**

The intrinsic activity of Compound I was firstly assessed as a topically active antiinflammatory agent by using a solution of Compound 140mg/ml in pure DMSO in a short-term exposure. When compared to the inhalation of DMSO alone, the inhalation of this formulation led to a significant decrease of BAL eosinophils (p<0.005), peribronchial inflammation scores (p<0.01), as well as bronchial hyperresponsiveness (p<0.05).

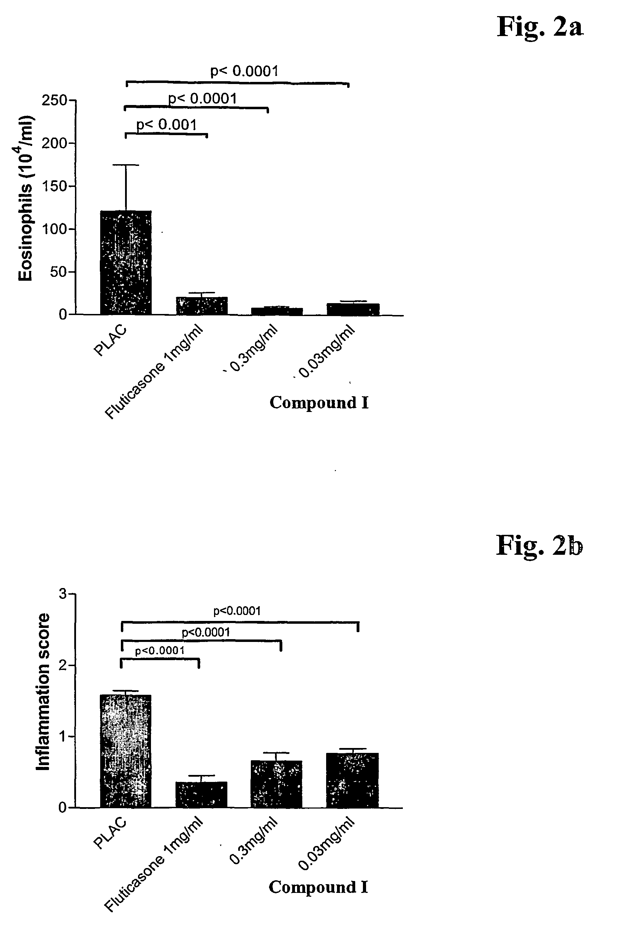

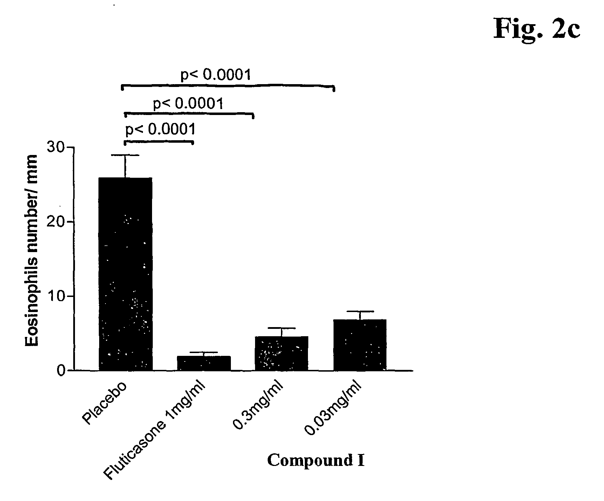

In the short-term exposure protocol, we assessed the effects of HP-$\beta$-CD Compound I complexes containing formulations on the airway inflammation and hyperresponsiveness. The effects of inhalation of Compound I- HP$\beta$CD complex containing formulations were compared with those of placebo (PBS) or fluticasone (1mg/ml) used as reference therapy. Inhalation of those formulations containing Compound I at doses of 0.03 and 0.3 mg/ml induced a significant decrease in eosinophilic inflammation in BAL in an extent comparable to that of fluticasone when compared to placebo (p<0.0001) (Figure 2a). Peribronchial inflammation scores were also lowered when compared to placebo (p<0.0001) (Figure 2b), as well as the tissue eosinophil infiltration score (p<0.01) (Figure 2c).

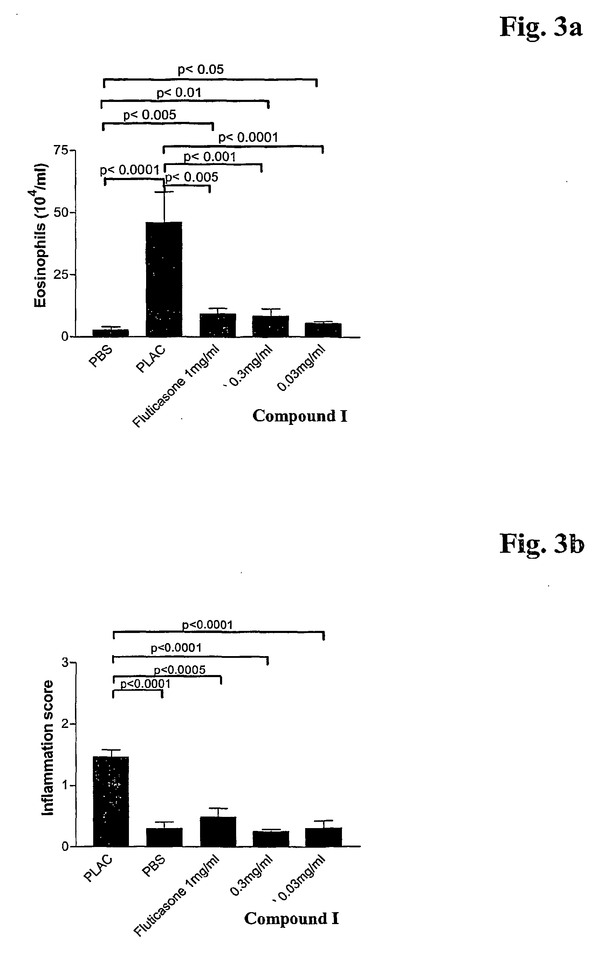

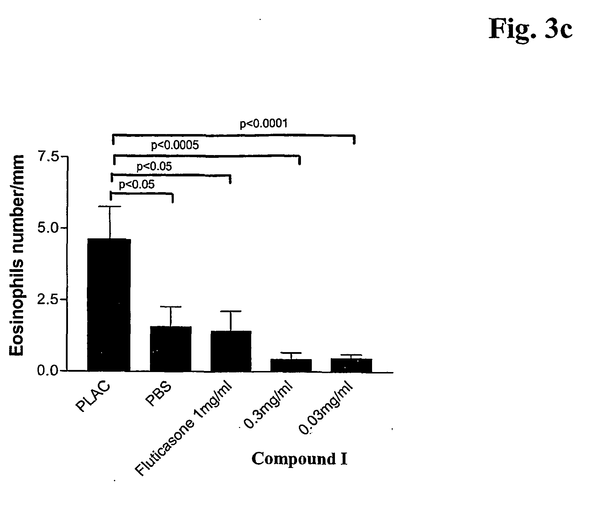

After long term allergen exposure, BAL eosinophilia was significantly decreased after treatment by inhalation of Compound I- HP$\beta$CD containing formulations (p<0.001) in the same extent as that of fluticasone (Figure 3a). The peribronchial inflammation score was also significantly decreased by inhalation of Compound I-HP$\beta$CD containing formulations as well as by fluticasone (p<0.0001) (Figure 3b). The tissue eosinophil infiltration score was also decreased after treatment by Compound I inhalation in an extent comparable to the fluticasone treated mice (p<0.01) (Figure 3c).

### Example 6

**Pharmacokinetic studies on the bioavailability**

Solutions for the pharmacokinetic studies were developed with a combination of HP-β-CD and L-lysine allowing a high Compound I concentration with a biocompatible pH value.

**Dosage form preparations**

The Compound I / HP-β-CD intravenous solution was obtained by dissolving Compound I (10 mg/ml) in a solution containing HP-β-CD (200 mM), L-lysine (20 mM) and water for injection. The osmolality (about 325 mOsmol/kg) and the pH (about 8.2) values of this solution are compatible with an intravenous injection. The solution was sterilized by passing through a sterile 0.20 μm cellulose acetate filter under aseptic conditions.

The Compound I / HP-β-CD oral solution was prepared by dissolving Compound I (15 mg/ml) in a solution containing HP-β-CD (200mM), L-lysine (50mM) and water.

The Compound I suspension was composed of Compound I (15 mg/ml), polysorbate 80 (0.1 mg/ml) as wetting agent, simaldrate (VEEGUM HV®, 1 % m/v) and methylcellulose (METHOCEL A400®, 0.4 % m/v) as viscosifying agents.

**Animal experimental protocol and drug administration**

Six healthy sheep (2 males and 4 females) ranging from 45 to 82 kg of body weight were used as experimental animals. During the test, the animals were fed and watered ad libitum.

The experimental study, which was realized following the scheme of Table 3, included a randomized two-way cross-over design for oral administration followed by an intravenous administration. A wash-out period of 3 weeks was allowed between each administration.

**Table 3:**

**Animal experimental design for administration of solutions and suspension containing Compound I**

| Sheep | 1st phase | 2nd phase | 3rd phase |
|---|---|---|---|
| 1 | Oral suspension | Oral solution | I.V. solution |
| 2 | Oral suspension | Oral solution | I.V. solution |
| 3 | Oral suspension | Oral solution | I.V. solution |
| 4 | Oral solution | Oral suspension | I.V. solution |
| 5 | Oral solution | Oral suspension | I.V. solution |
| 6 | Oral solution | Oral suspension | I.V. solution |

For the oral dosage forms, each animal received a Compound I dose equal to 15 mg / kg of body weight from both formulations. Sheep were weighed on the day of drug administration in order to adapt the dosage form volume. Blood samples were taken from jugular vein before and 0.25, 0.5, 1, 1.5, 2, 3, 4, 6, 8, 10, 12, 24, 28, 32, 48, 72, 96, 120, 144, 168 hours after oral administration.

For the intravenous dosage form, all six sheep received 5 mg of Compound I / kg of body weight. The solution was administered through the left jugular vein and blood samples were taken from the right jugular vein before and 5, 10, 15, 20, 30, 45 min, 1, 1.5, 2, 3, 4, 5, 6, 8, 10, 12, 24, 28, 32, 48, 72, 96, 120, 144, 168 h after starting the intravenous administration.

All blood samples were centrifuged and the serum were stored at -80°C until assayed.

**Bioanalysis method**

A fully automated method was developed for the LC determination of this compound in serum. Sample clean-up was performed by on-line coupling of a pre-column packed with restricted access material (RAM), namely LiChrospher RP-8 ADS (alkyl diol silica), to the analytical column by means of the column-switching technique. The ADS sorbents belong to the group of internal surface reversed-phase supports and have been applied successfully for the clean-up of biological samples prior to LC analysis (Yu, Z., and Westerlund, D., Chromatographia 44 (1997) 589-594; Hubert, Ph., et al., S. T. P. Pharma Pratiques 9 (1999) 160-180; Souverain, S., et al., Journal of Chromatography B 801 (2004) 141-156). The operating conditions are described in a previous paper (Chiap, P., et al., Journal of Chromatography B 817 (2005), 109-117). The method was fully validated according to a novel approach based on accuracy profiles taking into account the total measurement error (Hubert, P., et al., Analytica Chimica Acta 391 (1999) 135-148; Hubert, Ph., et al., S. T. P. Pharma Pratiques 13 (2003) 27-64; Hubert, Ph., et al., J Pharm Biomed. Anal. 36 (2004) 579-586.

For the bioanalytical study, the dosing range of the method had to be increased until 50 $\mu$g/ml due to high concentrations to be determined. A partial revalidation was performed and good results were obtained with respect to response function, trueness, precision, accuracy and linearity.

**Pharmacokinetics and statistical analysis**

For the intravenous administration study, the pharmacokinetic parameters were determined for each animal using a linear two-compartment model with first-order distribution and elimination (Boroujerdi, M., Pharmacokinetics, Principles and Applications. McGrow-Hill Companies, USA, 2002). The areas under the curve values (AUCs $_{0-168}$) were calculated by linear trapezoidal rule during the sampling period. The AUC extrapolated until infinite values (AUCs $_{0-\infty}$), the total body clearance values (Cl $_t$), the biologic half-life (T $_{1/2\beta}$) and the overall volume of distribution (Vd $_t$) were calculated using conventional equations associated with compartmental analysis (Boroujerdi, M., Pharmacokinetics, Principles and Applications, McGrow-Hill Companies, USA, 2002).

For the oral administration study, the pharmacokinetic parameters were determined, for each animal and for both suspension and solution, using a linear one-compartment model with first-order input and first-order output (Boroujerdi, M., Pharmacokinetics, Principles and Applications. McGrow-Hill Companies, USA, 2002). The AUCs $_{0-168}$ were calculated as described above by trapezoidal summation. The AUCs $_{0-\infty}$ were estimated by the following equation (equation 1):

$$AUC_{0-\infty} = C_0 \left( \frac{1}{K} - \frac{1}{k_a} \right)$$

**Equation 1**

where K and $k_a$ are respectively overall elimination rate constant and absorption rate constant and $C_o$ is the extrapolated concentration at the origin.

The maximum concentrations of drug in plasma ($C_{max}$) and the corresponding times ($T_{max}$) were determined for each animal by two different means: directly from the concentration-time graphs ($C_{max}$ experimental and $T_{max}$ experimental) and calculated using the following equations (equation 2 and 3) ($C_{max}$ calculated and $T_{max}$ calculated):

$$C_{max\ calculated} = C_0 \left( e^{-KT_{max}} - e^{-k_a T_{max}} \right)$$

**Equation 2**

$$T_{max\ calculated} = \frac{2.303}{k_a - K} \log \frac{k_a}{K}$$

**Equation 3**

Absolute bioavailability ($F_{absol}$) was evaluated using the following relation (equation 4):

$$F_{absol} = \frac{AUC_{oral} . D_{IV}}{AUC_{IV} . D_{oral}}$$

**Equation 4**

where D oral and D I.V. are the oral and I.V. administered drug quantities respectively.

All pharmacokinetic parameters are reported as means $\pm$ standard deviations except absolute bioavailability, calculated from average $AUC_{0-\infty}$.

Data were regarded as aberrant when the individual AUC value was higher or lower than mean $\pm$ 2 standard

deviations. Based on this, one sheep was excluded from the pharmacokinetic parameters determination after the oral solution administration and for statistical analysis.

The comparison of pharmacokinetic parameters for the two oral dosage forms has been performed with a two-way analysis of variance (two-way ANOVA). After log-transformation in order to normalize the distribution, the mean values of each calculated parameter were compared. Results were considered to be significant at the 5% critical level ($p < 0.05$).

**Pharmacokinetics of Compound I after intravenous administration**

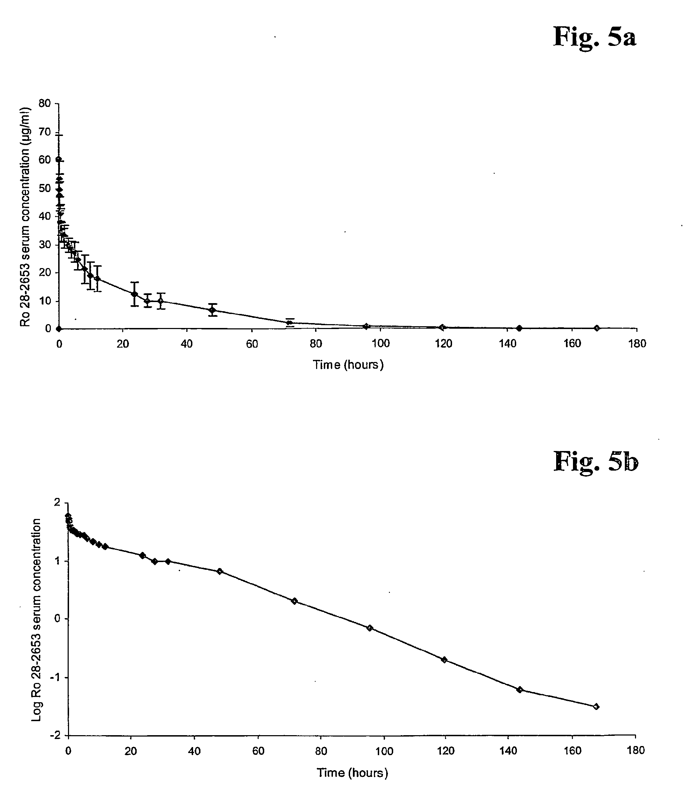

The mean Compound I serum concentration versus time curve obtained after a single administration of the intravenous solution (5 mg/kg) to sheep is reported in Fig. 5a. Fig 5b (logarithm of the mean Compound I serum concentration versus time curve) shows that the Compound I pharmacokinetics follow a two-compartment model. The different pharmacokinetic parameters calculated after this intravenous administration are listed in Table 4.

**Table 4:**

**Compound I pharmacokinetic parameters (mean $\pm$ S.D.) obtained after intravenous administration (5 mg/kg) to sheep (n = 6)**

|  | I.V. Solution |
|---|---|
| AUC $_{0-168h}$ ($\mu$g.h/ml) | 858,11 $\pm$ 211,58 |
| AUC $_{0-\infty}$ ($\mu$g.h/ml) | 858,87 $\pm$ 212,08 |
| Cl $_t$ (ml/h) | 358,76 $\pm$ 67,47 |
| Vd$_t$(1) | 8,18 $\pm$ 2,16 |
| T $_{1/2\beta}$ (h) | 15,76 $\pm$ 2,34 |

The distribution phase is short (about 30 minutes) showing that Compound I is rapidly distributed in the organism. The overall volume of distribution is small (about 8 liters) which indicates that Compound I distribution would be limited to extracellular fluids and that Compound I diffusion into tissues would not be very important. On the other hand, the Compound I biologic half-life is long (about 15.5 h) and, so, drug elimination is very slow. Considering its small distribution volume, the accumulation in the organism would not be caused by storage for example in fat but maybe by a strong binding with proteins or other components of plasma. The total body clearance value was also calculated and is around 358.5 ml/h.

**Pharmacokinetics of Compound I after oral administration of a suspension and a solution**

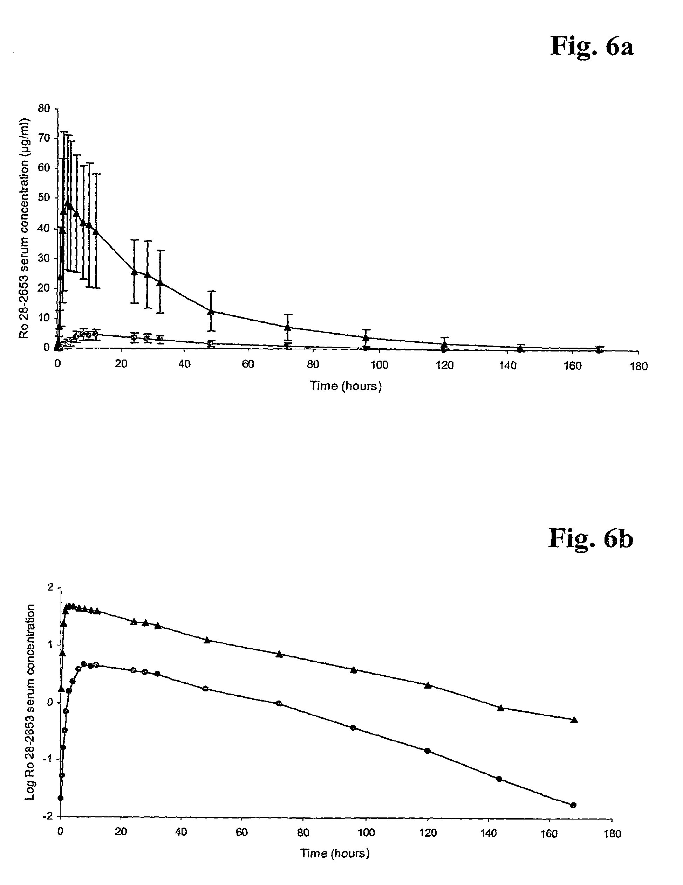

The mean serum concentration versus time profiles of Compound I obtained after oral administration of a single dose (15 mg/kg) of Compound I solution and suspension are shown in Fig. 6a. After logarithmic transformation of mean serum concentration, it seems that the pharmacokinetics after oral administration would follow a one-compartment model (Fig. 6b). The pharmacokinetic parameters are summarized in Table 5.

**Table 5:**

| Compound I pharmacokinetic parameters (mean $\pm$ S.D., except for F) obtained after oral administration (15 mg/kg) to sheep | | | |
|---|---|---|---|
|  | Oral Solution (n=5) | Suspension (n=6) | p-value (n=5) |
| AUC $_{0-168h}$ ($\mu$g.h/ml) | 1848,66 $\pm$ 854,97 | 208,94 $\pm$ 103,82 |  |
| AUC $_{0-\infty}$ ($\mu$g.h/ml) | 2070,13 $\pm$ 943,79 | 214,65 $\pm$ 103,04 | 0.0035 |
| C$_{max\ experimental}$ ($\mu$g/ml) | 51,84 $\pm$ 23,73 | 4,84 $\pm$ 1,95 | 0.0009 |
| C $_{max\ calculated}$ ($\mu$g/ml) | 56,85 $\pm$ 24,67 | 5,34 $\pm$ 2,24 | 0.0010 |
| T $_{max\ experimental}$ (h) | 3,59 $\pm$ 1,52 | 12,34 $\pm$ 5,99 | 0.0094 |
| T $_{max\ calculated}$ (h) | 3,98 $\pm$ 0,57 | 10,42 $\pm$ 3,01 | 0.0046 |
| F$_{absol}$ | 0,80 | 0,08 |  |

The serum concentrations of Compound I after administration of the solution are clearly higher than those obtained with an equal dose administered as a suspension. The absorption phase observed with the solution (about 4 h) is shorter than that achieved after administration of the suspension (about 10 h). It can also be seen that the pharmacokinetic parameters of the solution and the suspension are significantly different ($p < 0.05$) (Table 5). The mean Compound I serum peak concentrations are about 54 and 5 $\mu$g/ml after administration of the solution and the suspension respectively. $C_{max}$ of the solution is about 10 times higher than that of the suspension. A three times earlier $T_{max}$ is obtained with the solution (about 3.8h) than with the suspension (about 11h). The AUC values follow the same trend as do the $C_{max}$ values: the AUCs after administration of the solution are about 10-fold higher than those after administration of the suspension. Consequently, after comparison with the I.V. solution, the absolute bioavailability is much higher with the solution (80%) than with the suspension (8%).

**List of References**

Bergers, G., et al., Nat. Cell Biol. 2 (2000) 737-744

Boroujerdi, M., Pharmacokinetics, Principles and Applications, McGrow-Hill Companies, USA, 2002

Carmeliet, P., and Jain, R.K., Nature 407 (2000) 249-257

Carmeliet, P., et al., Nat. Genet. 17 (1997) 439-444

Cataldo, D.D., et al, Am. J. Pathol.161 (2002) 491-498

Chang, C., and Werb, D., Trends Cell Biol. 11 (2001) S37-43

Chiap, P., et al., Journal of Chromatography B 817 (2005), 109-117

Drummond, A.H., et al., Ann. N.Y. Acad. Sci. 878 (1999) 228-235

Egeblad, M., and Werb, Z., Nat. Rev. Cancer 2 (2002) 161-174

Fletcher, L., Nat. Biotechnol. 18 (2000) 1138-1139

Giavazzi, R., et al., Clin. Cancer Res. 4 (1998) 985-992

Grams, F., et al., Biol. Chem. 382 (2001) 1277-1285

Hamelmann, E., et al., Am. J Respir. Crit. Care Med. 156 (1997) 766-775

Higuchi, T., and Connors, K.A., Advances in Analytical Chemistry and Instrumentation 4 (1965) 117-212

Holmbeck, K., et al., Cell 99 (1999) 81-92

Hubert, P., et al., Analytica Chimica Acta 391 (1999) 135-148

Hubert, Ph., et al., J Pharm Biomed. Anal. 36 (2004) 579-586

Hubert, Ph., et al., S. T. P. Pharma Pratiques 9 (1999) 160-180

Hubert, Ph., et al., S. T. P. Pharma Pratiques 13 (2003) 27-64

Lund, L.R., et al., EMBO J. 18 (1999) 4645-4656

Manes, S., et al., J. Biol. Chem. 274 (1999) 6935-6945

Overall, C.M., and Lopez-Otin, C., Nat. Rev. Cancer 2 (2002) 657-672

Remington's Pharmaceutical Sciences, 16th ed., 1980, Mack Publishing Co., edited by Oslo et al.

Rohatagi, S., et al., J. Clin. Pharmacol. 43 (2003) 365-378

Shapiro, S.D., Curr. Opin. Cell Biol. 10 (1998) 602-608

Souverain, S., et al., Journal of Chromatography B 801 (2004) 141-156

Stack, M.S., and Gray, R.D., J. Biol. Chem. 264 (1989) 4277-4281

Szelenyi, I., et al., Drugs Today 36 (2000) 313-320

US Patent No. 6,110,924

US Patent No. 6,242,455

Vu, T.H., et al., Cell 93 (1998) 411-422

WO 01/25217

WO 97/23465

WO 98/58915

Yu, Z., and Westerlund, D., Chromatographia 44 (1997) 589-594

**Claims**

**1.** Use of a trioxopyrimidine compound having an inhibitory activity against MMP-1, MMP-2, MMP-3, MMP-9 and MMP-14 defined as

    a) an $IC_{50}$ value of less than 5 $\mu$M for MMP-2, MMP-9 and MMP-14 each;

b) a ratio of more than 100 for the $IC_{50}$ values of MMP-1:MMP-2, MMP-1: MMP-9, MMP-1:MMP-14; and
c) a ratio of more than 10 for the $IC_{50}$ values of MMP-3:MMP-2, MMP-3: MMP-9, MMP-3:MMP-14,
for the manufacturing of a medicament for the treatment of bronchial inflammatory diseases in a host mammal in need of such treatment;

**characterized in that** said trioxopyrimidine compound is selected from the group consisting of

5-Biphenyl-4-yl-5- [4-(4-nitro-phenyl)-piperazin-1-yl]pyrimidine-2,4,6-trione
5-(4-Phenoxy-phenyl)-5-(4-pyrimidin-2-yl-piperazin-1-yl)-pyrimidine-2,4,6-trione
5- [4-(4-Chloro-phenoxy)-phenyl]-5-(4-pyrimidin-2-yl-piperazin-1-yl)-pyrimidine-2,4,6-trione
5- [4-(3,4-Dichloro-phenoxy)-phenyl]-5-(4-pyrimidin-2-yl-piperazin-1-yl)-pyrimidine-2,4,6-trione
5- [4-(4-Bromo-phenoxy)-phenyl] -5-(4-pyrimidin-2-yl-piperazin-1-yl)-pyrimidine-2,4,6-trione.

2. Use according to claim 1, **characterized in that** the trioxopyrimidine compound is complexed with a water-soluble cyclodextrin.

**Patentansprüche**

1. Verwendung einer Trioxopyrimidinverbindung, die eine inhibitorische Aktivität gegenüber MMP-1, MMP-2, MMP-3, MMP-9 und MMP-14 aufweist, welche definiert sind durch

a) einen $IC_{50}$ Wert von weniger als 5 $\mu$M für jeweils MMP-2, MMP-9 und MMP-14;
b) ein Verhältnis von mehr als 100 für die $IC_{50}$ Werte von MMP-1:MMP-2, MMP-1:MMP-9, MMP-1 :MMP-14 und
c) ein Verhältnis von mehr als 10 für die $IC_{50}$ Werte von MMP-3:MMP-2, MMP-3:MMP-9, MMP-3:MMP-14,

zur Herstellung eines Medikaments für die Behandlung von inflammatorischen Bronchialkrankheiten bei einem Säuger, der einer solchen Behandlung bedarf;
**dadurch gekennzeichnet, dass** die Trioxopyrimidinverbindung ausgewählt ist aus der Gruppe bestehend aus

5-Biphenyl-4-yl-5-[4-(4-Nitro-Phenyl)-Piperazin-1-yl]Pyrimidin-2,4,6-trion
5-(4-Phenoxy-Phenyl)-5-(4-Pyrimidin-2-yl-Piperazin-1-yl)-Pyrimidin-2,4,6-trion
5-[4-(4-Chlor-Phenoxy)-Phenyl]-5-(4-Pyrimidin-2-yl-Piperazin-1-yl)-Pyrimidin-2,4,6-trion
5-[4-(3,4-Dichlor-Phenoxy)-Phenyl]-5-(4-Pyrimidin-2-yl-Piperazin-1-yl)-Pyrimidin-2,4,6-trion
5-[4-(4-Brom-Phenoxy)-Phenyl]-5-(4-Pyrimidin-2-yl-Piperazin-1-yl)-Pyrimidin-2,4,6-trion.

2. Verwendung nach Anspruch 1, **dadurch gekennzeichnet, dass** die Trioxopyrimidinverbindung mit einem wasserlöslichen Cyclodextrin komplexiert ist.

**Revendications**

1. Utilisation d'un composé trioxopyrimidine ayant une activité inhibitrice contre MMP-1, MMP-2, MMP-3, MMP-9 et MMP-14 définie comme

a) une valeur $IC_{50}$ inférieure à 5 $\mu$M pour MMP-2, MMP-9 et MMP-14 chacune ;
b) un ratio supérieur à 100 pour les valeurs $IC_{50}$ de MMP-1:MMP-2, MMP-1:MMP-9, MMP1-MMP-14 ; et
c) un ratio supérieur à 10 pour les valeurs $IC_{50}$ de MMP-3:MMP-2, MMP-3:MMP-9, MP-3:MMP-14,
pour la fabrication d'un médicament destiné au traitement des affections inflammatoires des bronches chez un mammifère hôte nécessitant ce type de traitement ;

**caractérisée en ce que** ledit composé trioxopyrimidine est choisi dans le groupe consistant en

5-biphényl-4-yl-5-[4-(4-nitro-phényl)pipérazine-1-yl]pyrimidine-2,4,6-trione
5-(4-phénoxy-phényl)-5-(4-pyrimidine-2-yl-pipérazine-1-yl)pyrimidine-2,4,6-trione
5- [4-(4-chloro-phénoxy)phényl]-5-(4-pyrimidine-2-yl-pipérazine-1-yl)pyrimidine-2,4,6-trione
5-[4-(3,4-dichloro-phénoxy)phényl]-5-(4-pyrimidine-2-yl-pipérazine-1-yl)pyrimidine-2,4,6-trione
5-[4-(4-bromo-phénoxy)phényl]-5-(4-pyrimidine-2-yl-pipérazine-1-yl)pyrimidine-2,4,6-trione.

**2.** Utilisation selon la revendication 1, **caractérisée en ce que** le composé trioxopyrimidine est complexé avec une cyclodextrine hydrosoluble.

**Fig. 1a**

**Fig. 1b**

**Fig. 2a**

**Compound I**

**Fig. 2b**

**Compound I**

**Fig. 2c**

**Fig. 3a**

**Fig. 3b**

Fig. 3c

Fig. 4

**Fig. 5a**

**Fig. 5b**

**Fig. 6a**

**Fig. 6b**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9723465 A
- WO 0125217 A
- EP 0204744 W
- US 6242455 B
- US 6110924 B
- WO 9858915 A
- US 6110924 A

**Non-patent literature cited in the description**

- **EGEBLAD, M. ; WERB, Z.** *Nat. Rev. Cancer,* 2002, vol. 2, 161-174
- **OVERALL, C.M. ; LOPEZ-OTIN, C.** *Nat. Rev. Cancer,* 2002, vol. 2, 657-672
- **HOLMBECK, K. et al.** *Cell,* 1999, vol. 99, 81-92
- **VU, T.H. et al.** *Cell,* 1998, vol. 93, 411-422
- **SHAPIRO, S.D.** *Curr. Opin. Cell Biol.,* 1998, vol. 10, 602-608
- **LUND, L.R. et al.** *EMBO J.,* 1999, vol. 18, 4645-4656
- **CARMELIET, P. et al.** *Nat. Genet.,* 1997, vol. 17, 439-444
- **CHANG, C. ; WERB, D.** *Trends Cell Biol.,* 2001, vol. 11, 37-43
- **MANES, S. et al.** *J. Biol. Chem.,* 1999, vol. 274, 6935-6945
- **BERGERS, G. et al.** *Nat. Cell Biol.,* 2000, vol. 2, 737-744
- **DONG, Z. et al.** *Cell,* 1997, vol. 88, 801-810
- **BROWN, P.D.** *Med. Oncol.,* 1997, vol. 14, 1-10
- **FLETCHER, L.** *Nat. Biotechnol.,* 2000, vol. 18, 1138-1139
- **DRUMMOND, A.H. et al.** *Ann. N.Y. Acad. Sci.,* 1999, vol. 878, 228-235
- **GIAVAZZI, R. et al.** *Clin. Cancer Res.,* 1998, vol. 4, 985-992
- **SZELENYI, I. et al.** *Drugs Today,* 2000, vol. 36, 313-320
- **ROHATAGI, S. et al.** *J. Clin. Pharmacol.,* 2003, vol. 43, 365-378
- **STACK, M.S. ; GRAY, R.D.** *J. Biol. Chem.,* 1989, vol. 264, 4277-4281
- **GRAMS, F. et al.** *Biol. Chem.,* 2001, vol. 382, 1277-1285
- GINA Workshop Report, Global Strategy for Asthma Management and Prevention. NIH Publication
- **FABBRI, L.M. ; HURD, S.S.** *Eur. Respir. J.,* 2003, vol. 22, 1-2
- Remington's Pharmaceutical Sciences. Mack Publishing Co, 1980
- **HIGUCHI, T. ; CONNORS, K.A.** *Advances in Analytical Chemistry and Instrumentation,* 1965, vol. 4, 117-212
- **GRAY, R.D.** *J. Biol. Chem.,* 1989, vol. 264, 4277-4281
- **HAMELMANN, E. et al.** *Am. J Respir. Crit. Care Med.,* 1997, vol. 156, 766-775
- **CATALDO, D.D. et al.** *Am. J. Pathol.,* 2002, vol. 161, 491-498
- **YU, Z. ; WESTERLUND, D.** *Chromatographia,* 1997, vol. 44, 589-594
- **HUBERT, PH. et al.** *S. T. P. Pharma Pratiques,* 1999, vol. 9, 160-180
- **SOUVERAIN, S. et al.** *Journal of Chromatography,* 2004, vol. 801, 141-156
- **CHIAP, P. et al.** *Journal of Chromatography,* 2005, vol. 817, 109-117
- **HUBERT, P. et al.** *Analytica Chimica Acta,* 1999, vol. 391, 135-148
- **HUBERT, PH. et al.** *S. T. P. Pharma Pratiques,* 2003, vol. 13, 27-64
- **HUBERT, PH. et al.** *J Pharm Biomed. Anal.,* 2004, vol. 36, 579-586
- **BOROUJERDI, M.** Pharmacokinetics, Principles and Applications. McGrow-Hill Companies, 2002
- **CARMELIET, P. ; JAIN, R.K.** *Nature,* 2000, vol. 407, 249-257